# EUROPEAN PATENT SPECIFICATION

(11) **EP 4 608 804 B1**
(45) Date of publication and mention of the grant of the patent: **21.10.2026**
(21) Application number: 23841429.6
(22) Date of filing: 06.12.2023
(51) Int. Cl.: C07C 279/26, C07C 309/04, A61K 8/43, A61Q 11/00

(54) **NOVEL CHLORHEXIDINE SALTS AND RELATED COMPOSITIONS AND METHODS**
NEUE CHLORHEXIDINSALZE UND ZUGEHÖRIGE ZUSAMMENSETZUNGEN UND VERFAHREN
NOUVEAUX SELS DE CHLORHEXIDINE ET COMPOSITIONS ET PROCÉDÉS ASSOCIÉS

(30) Priority: 06.12.2022 US 202263430609 P
(43) Date of publication of application: 03.09.2025
(73) Proprietor: Colgate-Palmolive Company, New York, NY 10022 (US)
(72) Inventor: DUBOVOY, Viktor, Basking Ridge, New Jersey 07920 (US); CHEN, Dailin, Guangzhou, Guangdong 510730 (CN); HAO, Zhigang, Bridgewater, New Jersey 08807 (US); HE, Xing, Guangzhou, Guangdong 510730 (CN); PAN, Long, Somerset, New Jersey 08873 (US); CHENG, Chi-Yuan, Hillsborough, New Jersey 08844 (US); WANG, Yu, Edison, New Jersey 08817 (US); SALERNO, Jake, New York, New York 10022 (US); BRINZARI, Tatiana, Franklin Park, New Jersey 08823 (US)
(74) Representative: Sonnenhauser, Thomas Martin
(86) International application number: PCT/US2023/082757
(87) International publication number: WO 2024/123928

(56) References cited:
- WO-A1-2022/251439
- US-A1- 2004 137 065

## Description

### FIELD

This application relates to a novel complex of lauryl sulfate and chlorhexidine, which provides enhanced chlorhexidine stability and anti-bacterial activity.

### BACKGROUND

Chlorhexidine (CHX) is widely used in mouthwashes to treat gingivitis, to help prevent dental plaque and caries, and to protect against infections following oral surgery and tooth extraction. Usually, chlorhexidine is provided in the form of chlorhexidine gluconate (CHG). Chlorhexidine can also be used for skin disinfection before surgery, sterilization of surgical instruments, cleaning wounds, treating yeast infections of the mouth, and keeping urinary catheters from blocking. Chlorhexidine, however, can degrade during storage, and the formulation can affect its antibacterial efficacy. Improved formulations of chlorhexidine, providing enhanced stability and antibacterial efficacy, are needed.
US 2004/1371065 A1 discloses an antibiotic polymer combination/antibiotics polymer combination, comprising (a) one or more antibiotic salts, which are sparingly soluble in water, from the groups comprising aminoglycoside antibiotics, lincosamide antibiotics, tetracycline antibiotics, glycopeptide antibiotics, quinolone antibiotics and chlorhexidine, and optionally (b) an antibiotic, which is readily soluble in water, from the groups comprising aminoglycoside antibiotics, lincosamide antibiotics, β-lactam antibiotics and tetracycline antibiotics, and optionally (c) one or more organic ancillary substances suspended in a homogenous polymer mixture to form a suspension wherein the homogeneous polymer mixture comprises one or more hydrophobic, nonionic polymers from the groups comprising poly(vinyl chloride), post-chlorinated poly(vinyl chloride), poly(vinylidene chloride), poly(vinyl fluoride), poly(vinylidene fluoride) and copolymers comprising vinyl chloride and one or more nonionic monomers, and which comprises one or more hydrophilic polymers from the groups comprising polyethers, and wherein the suspension forms a composite.
WO 2022/251439 A1 discloses an oral care composition comprising: (i) an effective amount of a bisbiguanide in free or orally acceptable salt form; (ii) an effective amount of a nonionic cellulose ether; (iii) an effective amount of a zwitterionic surfactant; and (iv) water.

### SUMMARY

It is surprisingly found that a novel complex of chlorhexidine lauryl sulfate (CHX-LS, also sometimes referred to herein as chlorhexidine dodecyl sulfate or CHX-DS) provides enhanced chlorhexidine stability and anti-bacterial activity, for example in an oral care composition.

### DRAWINGS

Figure 1 depicts NMR results of the CHX-LS crystal dissolved in DMSO.
Figure 2 shows FTIR absorption spectrum of the CHX-DS (a.k.a. CHX-LS) crystal prepared in this work in comparison to those of the reference materials of SDS (a.k.a. SLS) and CHG (chlorhexidine gluconate) is shown in Figure 2 (spectra offset for clarity)
Figure 3 depicts two alternative three-dimensional structures of the salt.
Figure 4 depicts methyl mercaptan measurement by gas chromatography to evaluate VSC reduction efficacy.

### DETAILED DESCRIPTION

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight relative to the total composition. The amounts given are based on the active weight of the material.

As is usual in the art, the compositions described herein are sometimes described in terms of their ingredients, notwithstanding that the ingredients may disassociate, associate, or react in the formulation. Ions, for example, are commonly provided to a formulation in the form of a salt, which may dissolve and disassociate in aqueous solution. It is understood that the invention encompasses both the mixture of described ingredients and the product thus obtained.

It is understood that all ingredients in the compositions described herein are safe and palatable in the relevant concentrations for oral administration as a mouthwash.

As described in the Examples, chlorhexidine lauryl sulfate (CHX-LS) is synthesized and characterized via single-crystal X-ray diffraction (SC-XRD), 1H nuclear magnetic resonance (NMR), 1H nuclear Overhauser effect spectroscopy (NOESY), and attenuated total reflectance Fourier-transform infrared spectroscopy (ATR-FTIR). The solid-state structure comprises a 1:2 stoichiometric ratio of chlorhexidine cation [C22H30Cl2N10]2+ to dodecyl sulfate anion [C12H25SO4]-. CHX-LS exhibits broad-spectrum antibacterial activity and demonstrates superior efficacy to reduce bacteria generated volatile sulfur compounds (VSCs) as compared to chlorhexidine gluconate (CHG). Surprisingly, whereas anionic surfactants such as sodium lauryl sulfate can interfere with the activity of CHX, providing the CHX in a stable complex with lauryl sulfate appears to enhance its activity.

The disclosure provides in one embodiment, a solid salt of chlorhexidine lauryl sulfate wherein the solid salt of chlorhexidine lauryl sulfate is: (i) in crystalline form; or (ii) in amorphous form, as defined in the claims.

Preferably, the molar ratio between chlorhexidine (CHX) and lauryl sulfate (LS) is 1:2.

The solid salt of chlorhexidine lauryl sulfate may have a structure (a) wherein two LS anions are each paired with a protonated guanidinium moiety of a CHX molecule, or (b) wherein two LS molecules are both paired with a single diprotonated guanidinium moiety of a CHX molecule. The solid salt of chlorhexidine lauryl sulfate can have a structure corresponding to Structure A or Structure B:

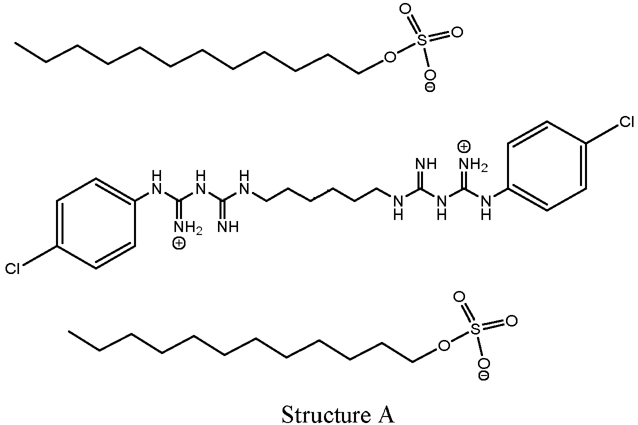

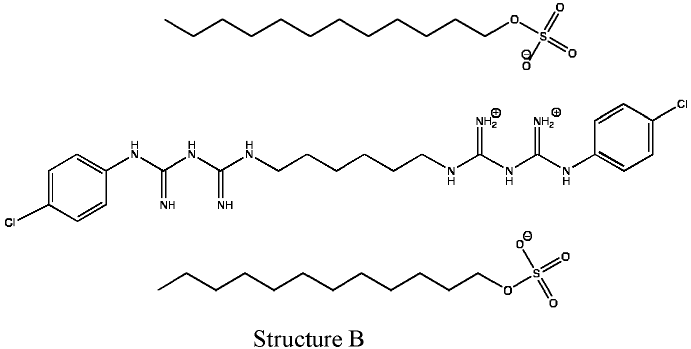

The solid salt of chlorhexidine lauryl sulfate may have a structure corresponding to Structure A.

In another embodiment, the disclosure provides an oral care composition comprising chlorhexidine lauryl sulfate wherein the molar ratio between chlorhexidine (CHX) and lauryl sulfate (LS) is 1:4 to 1:1, or preferably 1:2.

In the oral care composition, the chlorhexidine lauryl sulfate can be the solid salt of chlorhexidine lauryl sulfate.

In the oral care composition, the molar ratio between chlorhexidine (CHX) and lauryl sulfate (LS) can be approximately 1:2.

Optionally, in the oral care composition, the chlorhexidine lauryl sulfate is in crystalline form. Optionally, in the oral care composition, the chlorhexidine lauryl sulfate is in amorphous form. In the oral care composition, the chlorhexidine lauryl sulfate may have a structure (a) wherein two LS molecules are each paired with different guanidinium moiety of a CHX molecule, or (b) wherein two LS molecules are both paired with a single guanidinium moiety of a CHX molecule. The chlorhexidine lauryl sulfate may have a structure corresponding to Structure A, or the chlorhexidine lauryl sulfate may have a structure corresponding to or Structure B. The chlorhexidine lauryl sulfate may have a structure corresponding to Structure A.

The chlorhexidine lauryl sulfate can be added to the other formulation excipients as a pre-formed solid salt, e.g., the solid salt of chlorhexidine lauryl sulfate.

The chlorhexidine lauryl sulfate can be added to the formulation excipients as a pre-formed solid salt.

In the oral care composition, the chlorhexidine lauryl sulfate can be undissolved or partially undissolved.

The oral care composition may be a mouthwash; a nonabrasive gel; or a toothpaste or gel comprising abrasives.

The oral care composition can further comprise flavorings, sweeteners, humectants, and surfactants.

The oral care composition can be a mouthwash further comprising flavorings, sweeteners, humectants, and surfactants.

The oral care composition can be a toothpaste further comprising flavorings, sweeteners, humectants, surfactants, and abrasives.

The oral care composition can comprise one or more nonionic surfactants.

The nonionic surfactant may comprise a fatty acid moiety and a polyethylene glycol moiety. The nonionic surfactant may be selected from PEG-40 sorbitan diisostearate and PEG-40 hydrogenated castor oil.

The oral care composition may comprise a sweetener, e.g., a nonsaccharide sweetener, e.g., sodium saccharine.

The oral care composition may comprise a humectant, e.g., selected from propylene glycol, glycerol, sorbitol, and combinations thereof.

The oral care composition may comprise glycerol.

The oral care composition may comprise cetyl pyridinium chloride, e.g., in an amount of 0.01% to 0.05%, e.g., about 0.015% by weight.

The oral care composition may comprise a fluoride ion source, e.g., selected from sodium fluoride, stannous fluoride, sodium monofluorophosphate, amine fluorides (e.g. olaflur and/or dectaflur), and combinations thereof, e.g., in an amount to provide 200 to 500 ppm fluoride, e.g., about 225 ppm fluoride in a mouthwash, or to provide 1000 to 5000 ppm fluoride, e.g., about 1450 ppm fluoride in a toothpaste.

In the oral care composition, the concentration of chlorhexidine lauryl sulfate can be 0.05% to 0.4% by weight. The concentration of chlorhexidine lauryl sulfate can be0.05% to 0.25% by weight. The concentration of chlorhexidine lauryl sulfate can be about 0.25% by weight.

The concentration of chlorhexidine lauryl sulfate is 0.1% to 0.2% by weight.

The oral care composition may comprise at least 70% water by weight.

The oral care composition may comprise 5% to 25% humectant by weight.

Thethe oral care composition can be in the form of a mouthwash.

The oral care composition can comprise
0.1% to 0.15% chlorhexidine lauryl sulfate;
0% to 15% ethanol;
5% to 25% humectant selected from propylene glycol, glycerol, sorbitol, and combinations thereof;
0% to 0.02% cetyl pyridinium chloride;
0.1% to 1% nonionic surfactant, e.g., selected from PEG-40 sorbitan diisostearate, PEG-40 hydrogenated castor oil, and combinations thereof;
flavoring; coloring; and water;
wherein all percentage amounts are by weight of the composition.

The oral care composition can be free of ethanol.

The oral care composition can be free of chlorhexidine gluconate.

The oral care composition can be free of ingredients that are not orally acceptable, e.g., free of materials that are not safe and/or not palatable at the relevant concentrations for use in an oral care formulation, such as a mouthwash or a toothpaste.

The oral care composition can exhibit improved chlorhexidine stability upon aging in a 60 °C oven for at least 3 weeks, e.g., at least 6 weeks, relative to an aqueous formulation comprising the same amount of chlorhexidine in the form of chlorhexidine gluconate rather than chlorhexidine lauryl sulfate.

As defined in the claims, the oral care composition is for use in treating gingivitis, prophylaxis of dental plaque and caries, treating yeast infections of the mouth, and/or protecting against infections following oral surgery and tooth extraction.

As defined in the claims, the oral care composition is for use in reducing halitosis and/or reducing volatile sulfur compounds in the mouth.

The oral care composition can be used as an antimicrobial.

Preferably, the oral care composition is for use in treating gingivitis, prophylaxis of dental plaque and caries, treating yeast infections of the mouth, protecting against infections following oral surgery and tooth extraction, reducing halitosis and/or reducing volatile sulfur compounds in the mouth and or treating or reducing harmful bacteria in the mouth, and/or in a method for treating or reducing harmful bacteria in the mouth, which comprises administering an oral care composition comprising chlorhexidine lauryl sulfate wherein the molar ratio between chlorhexidine (CHX) and lauryl sulfate (LS) is 1:4 to 1:1, e.g., approximately 1:2, e.g., administering an oral care composition as described herein, to the oral cavity of a subject in need thereof, e.g., one, twice, or three times daily.

For example, in any one embodiment of treating gingivitis, prophylaxis of dental plaque and caries, treating yeast infections of the mouth, protecting against infections following oral surgery and tooth extraction, and/or reducing halitosis, the oral care composition is a mouthwash, which may be administered to the subject in need thereof twice daily by rinsing for approximately 30 seconds, morning and evening after tooth brushing, in a dosage of about 15 ml of undiluted mouthwash. The patient should be instructed to not rinse with water, or other mouthwashes, brush teeth, or eat immediately after using the mouthwash. The mouthwash is not intended for ingestion and should be expectorated after rinsing.

As defined in the claims, the disclosure provides a disinfectant composition comprising chlorhexidine lauryl sulfate, water, and alcohol (e.g., ethanol, isopropyl alcohol, and mixtures thereof), for example a composition comprising 1% to 5% chlorhexidine lauryl sulfate, 65% to 80% isopropyl alcohol, and water, wherein the molar ratio between chlorhexidine (CHX) and lauryl sulfate (LS) is 1:4 to 1:1, e.g., approximately 1:2; for example wherein the chlorhexidine lauryl sulfate can be the solid salt of chlorhexidine lauryl sulfate, e.g., wherein disinfectant composition is formed by mixing any of the solid salt of chlorhexidine lauryl sulfate with water and other excipients. As defined in the claims, the disinfectant composition is for use as a skin disinfectant before surgery and/or cleaning a wound. The disclosure further provides a method for sterilization of surgical or dental instruments comprising administering the disinfectant composition to the instruments, as defined in the claims.

In a further embodiment, the disclosure provides a method of stabilizing chlorhexidine in an aqueous formulation, e.g., in the oral care composition or in the disinfectant composition, comprising adding the solid salt of chlorhexidine lauryl sulfate to the aqueous solution, as defined in the claims.

As used herein, an "oral care composition" such as the mouthwashes and toothpastes of the disclosure refers to a composition for which the intended use includes oral care, oral hygiene, and/or oral appearance, or for which the intended method of use comprises administration to the oral cavity and refers to compositions that are palatable and safe for topical administration to the oral cavity, and for providing a benefit to the teeth and/or oral cavity. The term "oral care composition" thus specifically excludes compositions which are highly toxic, unpalatable, or otherwise unsuitable for administration to the oral cavity. In some embodiments, an oral care composition is not intentionally swallowed, but is rather retained in the oral cavity for a time sufficient to affect the intended utility. The oral care compositions as disclosed herein may be used in nonhuman mammals such as companion animals (e.g., dogs and cats), as well as by humans. In some embodiments, the oral care compositions as disclosed herein are used by humans. In some embodiments, the disclosure provides mouthwash formulations. In some embodiments, the disclosure provides toothpaste formulations.

As used herein, "nonionic surfactant" generally refers to compounds produced by the condensation of alkylene oxide groups (hydrophilic in nature) with an organic hydrophobic compound which may be aliphatic or alkyl-aromatic in nature. Examples of suitable nonionic surfactants include poloxamers (sold under trade name PLURONIC¬Æ), polyoxyethylene, polyoxyethylene sorbitan esters (sold under trade name TWFFNS¬Æ), Polyoxyl 40 hydrogenated castor oil, fatty alcohol ethoxylates, polyethylene oxide condensates of alkyl phenols, products derived from the condensation of ethylene oxide with the reaction product of propylene oxide and ethylene diamine, ethylene oxide condensates of aliphatic alcohols, alkyl polyglycosides (for example, fatty alcohol ethers of polyglycosides, such as fatty alcohol ethers of polyglucosides, e.g., decyl, lauryl, capryl, caprylyl, myristyl, stearyl and other ethers of glucose and polyglucoside polymers, including mixed ethers such as capryl/caprylyl (C8-10) glucoside, coco (C8-16) glucoside, and lauryl (C12-16) glucoside), long chain tertiary amine oxides, long chain tertiary phosphine oxides, long chain dialkyl sulfoxides, and mixtures of such materials.

In some embodiments, the nonionic surfactant comprises amine oxides, fatty acid amides, ethoxylated fatty alcohols, block copolymers of polyethylene glycol and polypropylene glycol, glycerol alkyl esters, polyoxyethytene glycol octylphenol ethers, sorbitan alkyl esters, polyoxyethylene glycol sorbitan alkyl esters, and mixtures thereof. Examples of amine oxides include, but are not limited to, laurylamidopropyl dimethylamine oxide, myristylamidopropyl dimethylamine oxide, and mixtures thereof. Examples of fatty acid amides include, but are not limited to, cocomonoethanolamide, lauramide monoethanolamide, cocodiethanolamide, and mixtures thereof. In certain embodiments, the nonionic surfactant is a combination of an amine oxide and a fatty acid amide. In certain embodiments, the amine oxide is a mixture of laurylamidopropyl dimethylamine oxide and myristylamidopropyl dimethylamine oxide. In certain embodiments, the nonionic surfactant is a combination of lauryl/myristylamidopropyl dimethylamine oxide and cocomonoethanolamide. In certain embodiments, the nonionic surfactant is present in an amount of 0.01 to 5.0%, 0.1 to 2.0%, 0.1 to 0.6%, 0.2 to 0.4%, about 0.2%, or about 0.5%.

Mouthwashes frequently contain significant levels of ethanol, which is often needed to solubilize essential oils and to prevent bacterial contamination. High levels of ethanol may be undesirable, because in addition to the potential for abuse by ingestion, the ethanol may exacerbate conditions like xerostomia. Accordingly, in some embodiments, the oral care compositions can be substantially free of ethanol, e.g., contain less than 1% ethanol.

Humectants can enhance the viscosity, mouthfeel, and sweetness of the product, and may also help preserve the product from degradation or microbial contamination. Suitable humectants include edible polyhydric alcohols such as glycerin, sorbitol, xylitol, propylene glycol as well as other polyols and mixtures of these humectants. Sorbitol may in some cases be provided as a hydrogenated starch hydrolysate in syrup form, which comprises primarily sorbitol (the product if the starch were completely hydrolyzed to glucose, then hydrogenated), but due to incomplete hydrolysis and/or presence of saccharides other than glucose, may also include other sugar alcohols such mannitol, maltitol, and longer chain hydrogenated saccharides, and these other sugar alcohols also function as humectants in this case. In some embodiments, humectants are present at levels of 5% to 25%, e.g., 15% to 20% by weight.

Flavorings may include extracts or oils from flavorful plants such as peppermint, spearmint, cinnamon, wintergreen, and combinations thereof, cooling agents such as menthol, methyl salicylate, and commercially available products such as OptaCool from Symrise, as well as sweeteners, which may include polyols (which also function as humectants), saccharin, acesulfame, aspartame, neotame, stevia and sucralose.

Toothpastes, including abrasive gels, may comprise one or more abrasives, e.g., silica abrasives or calcium abrasives, e.g., calcium carbonate dicalcium phosphate, or calcium pyrophosphate. For example, toothpaste compositions disclosed herein may include silica abrasives, and may comprise additional abrasives, e.g., a calcium phosphate abrasive, e.g., tricalcium phosphate (Ca₃(PO₄)₂), hydroxyapatite (Ca₁₀(PO₄)₆(OH)₂), or dicalcium phosphate dihydrate (CaHPO₄ • 2H₂O, also sometimes referred as DiCal) or calcium pyrophosphate; calcium carbonate abrasive; or abrasives such as sodium metaphosphate, potassium metaphosphate, aluminum silicate, calcined alumina, bentonite or other siliceous materials, or combinations thereof. These abrasives, generally have an average particle size ranging between about 1 and about 30 microns, about between 5 and about 15 microns. These particulate silica abrasives are distinct from colloidal silica thickeners.

Toothpastes may further comprise an anticalculus (tartar control) agent. Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. The toothpaste thus may comprise phosphate salts. In particular embodiments, these salts are alkali phosphate salts, i.e., salts of alkali metal hydroxides or alkaline earth hydroxides, for example, sodium, potassium or calcium salts. "Phosphate" as used herein encompasses orally acceptable mono- and polyphosphates, for example, P₁₋₆ phosphates, for example monomeric phosphates such as monobasic, dibasic or tribasic phosphate; dimeric phosphates such as pyrophosphates; and multimeric phosphates, e.g., sodium hexametaphosphate. In particular examples, the selected phosphate is selected from alkali dibasic phosphate and alkali pyrophosphate salts, e.g., selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, and mixtures of any of two or more of these. In a particular embodiment, for example the compositions comprise a mixture of tetrasodium pyrophosphate (Na₄P₂O₇), calcium pyrophosphate (Ca₂P₂O₇), and sodium phosphate dibasic (Na₂HPO₄), e.g., in amounts of ca. 3-4% of the sodium phosphate dibasic and ca. 0.2-1% of each of the pyrophosphates. In another embodiment, the compositions comprise a mixture of tetrasodium pyrophosphate (TSPP) and sodium tripolyphosphate (STPP)(Na₅P₃O₁₀), e.g., in proportions of TSPP at about 1-2% and STPP at about 7% to about 10%. Such phosphates are provided in an amount effective to reduce erosion of the enamel, to aid in cleaning the teeth, and/or to reduce tartar buildup on the teeth, for example in an amount of 2-20%, e.g., ca. 5-15%, by weight of the composition.

The oral care compositions disclosed herein may also include additional polymers to adjust the viscosity of the formulation or enhance the solubility of other ingredients and/or to form a gel. Such additional polymers include polysaccharides (e.g., cellulose derivatives, for example carboxymethyl cellulose, or polysaccharide gums, for example xanthan gum or carrageenan gum), and polyvinyl pyrrolidone. Acidic polymers, for example polyacrylate gels, may be provided in the form of their free acids or partially or fully neutralized water soluble alkali metal (e.g., potassium and sodium) or ammonium salts. Silica thickeners, which form polymeric structures or gels in aqueous media, may be present. Note that these silica thickeners are physically and functionally distinct from the particulate silica abrasives also present in some compositions, as the silica thickeners are very finely divided and provide little or no abrasive action. Other thickening agents are carboxyvinyl polymers, carrageenan, hydroxyethyl cellulose and water-soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate can also be used as component of the thickening composition to further improve the composition's texture. In certain embodiments, thickening agents in an amount of 0.5% to 5.0% by weight of the total composition are used.

Other ingredients which may optionally be included in compositions include hyaluronic acid, green tea, ginger, sea salt, coconut oil, turmeric, white turmeric (white curcumin), grape seed oil, ginseng, monk fruit, vitamin E, basil, chamomile, pomegranate, aloe vera, and charcoal. Any of such ingredients may be present in an amount from 0.01% to 2% by weight of the composition, e.g., 0.01 to 1%, or 0.01 to 0.5%, or 0.01 to 0.1%.

As used herein, "orally acceptable" refers to a material that is safe and palatable at the relevant concentrations for use in an oral care formulation, such as a mouthwash.

Unless stated otherwise, all percentages of composition components given in this specification are by weight based on a total composition or formulation weight of 100%.

It is understood that, in certain cases, an ingredient may perform multiple functions. For example, polyethylene glycol may affect the viscosity of the product, but may also act as a humectant.

The compositions and formulations as provided herein are described and claimed with reference to their ingredients, as is usual in the art. As would be evident to one skilled in the art, the ingredients may in some instances react with one another, so that the true composition of the final formulation may not correspond exactly to the ingredients listed. Thus, it should be understood that the invention extends to the product of the combination of the listed ingredients.

### EXAMPLES

### Example 1 - Characterization of CHX-LS Salt

*CHG and CHG-SLS mixture aging study:* 2 gram of 20% CHG solution is used as control and SLS is added in a 1:2 molar of SLS:CHG into another 2 gram of 20% CHG solution. Both aqueous solutions are placed in a 60 °C oven and aged for 3 and 6 weeks.

Sample preparation for CHX degradation analysis by using GCMS analysis: Both aged solutions are taken from the 60 °C oven. They are diluted at 1:3.5 ratios with methanol. Both solutions are filtrated with a 0.20 um PTFE filter, respectively, then they are diluted again at a 1:10 dilution ratio with methanol and then transferred into a ROBO autosampler vials for GC-MS analysis. The gas chromatography system 6890N with 5972 MS detector plus Gerstel MPS-2 autosampler (Agilenet Technologies, Santa, Clara, CA, USA) is used for detection of CHX degradation products. Separation is accomplished using GC column of HP-5MS (30m x 0.25mm x 0.25µm, length x inside diameter x film thickness, Agilent Technologies). The 1 µL sample is injected with the splitless mode. The oven temperature is initially held at 80 °C for 1 min. Thereafter the temperature is raised at 6 °C/min until 300 °C and held for 2.33 min. Total running time is 40 minutes. Helium is used as the carrier gas and delivered at a constant flow rate at 1 mL/min (the pressure at 9.38 psi (646.7 mbar) and velocity at 37 cm/sec). The injector temperature is set at 250 °C and the interface temperature between GC oven and MS detector is250 °C. The MS detectors are tuned with the standard spectrum autotune, and the MS data (total ion chromatogram, TIC) are acquired with the full scan mode (m/z of 45 - 550 at a scan rate of 3 scan/sec using the electron ionization (EI) mode with an electron energy of 70 eV. The MS source temperature is 230 °C and quat temperature is 150 °C.

| | Peak integration for CHX degradation product at 3 weeks | % degradation product in CHG-SLS relative to CHG at 3 weeks | Peak integration for CHX degradation product at 6 weeks | % degradation product relative to CHG at 6 weeks |
|---|---|---|---|---|
| CHG | 523490 | | 7560037 | |
| CHG-SLS | 21514 | 4.11% | 1587759 | 21.00% |

The relative degradation content from CHG-SLS aged at 60°C in 3 weeks is only 4% of that seen in the CHG solution control. Even aged at 60°C for 6 weeks, the degradation content in CHG-SLS mixture solutions is only about 21% relative the CHG placebo solution. Those results clearly indicated SLS can protect CHX degradation. At same time, a novel CHX-LS complex crystal is obtained from the CHG-SLS mixture.

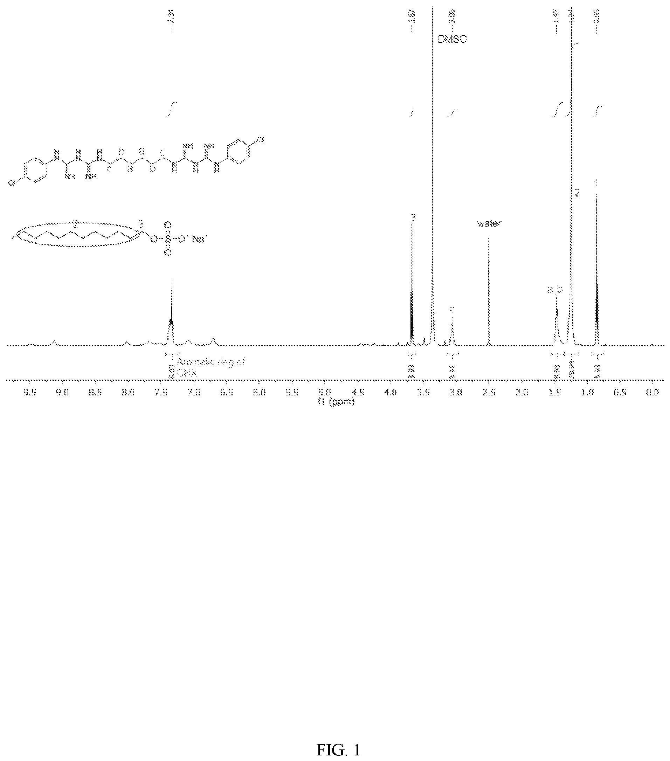

*NMR experiment:* The crystal sample is fully dissolved in deuterated DMSO and prepared at 0.1wt%. ¹H NMR spectra are conducted using Bruker spectrometer operating at proton frequency of 500.13 MHz equipped with a double resonance cryoprobe. The sample temperatures are controlled at 25°C. The spectrum is collected using a single pulse with a 30°C pulse angle, 1 second acquisition time, 5 second recycle delay, and a sweep width of 12 ppm. The number of scans is 32, required ~10 min for each spectral collection. The ¹H NMR spectral signals (Figure 1) can be assigned to the structure of CHX and LS. Based on the peak integral, the stoichiometric ratio between CHX and LS is 1:2.

*X-Ray experiment:* Chlorhexidine lauryl sulfate (CHX-LS), comprising of chlorhexidine and sodium lauryl sulfate (sometimes referred to as sodium dodecyl sulfate) is synthesized and characterized via single crystal X-ray diffraction measurements indicating a stoichiometry of [C₂₂H₃₂N₁₀Cₗ₂].[(C₁₂H₂₅O₄S)₂], wherein the molecules are arranged in a 1:2 ratio, with a doubly protonated chlorhexidine cation and two dodecyl sulfate anions.

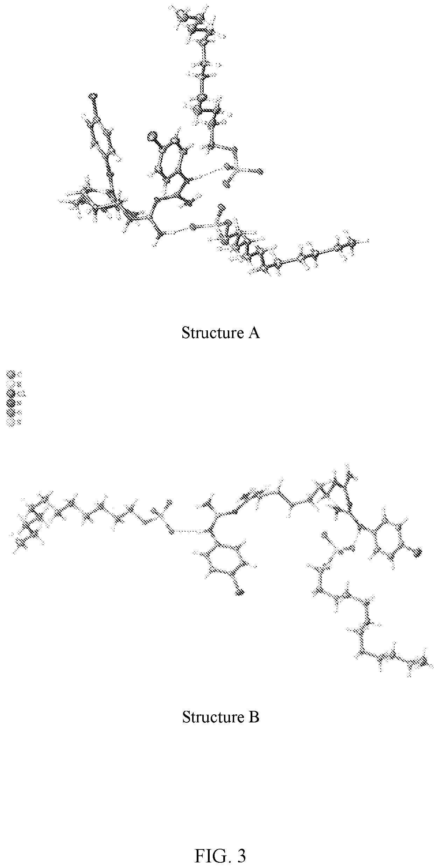

Crystals of CHX-LS suitable for X-ray crystallography are isolated under the microscope and the X-ray diffraction data are collected using Bruker D8 Venture PHOTON 100 CMOS system equipped with a Cu Kα INCOATEC ImuS micro-focus source (λ = 1.54178 Å). The data is collected at 100 K. Indexing is performed using APEX3 (Difference Vectors method). Data integration and reduction are performed using SaintPlus 6.01. Absorption correction is performed by multi-scan method implemented in SADABS. Space group is determined using XPREP implemented in APEX3. The structure is solved using SHELXT (direct methods) and is refined using SHELXL-2017 (full-matrix least-squares on F2 ) through OLEX2 interface program. All non-hydrogen atoms are refined anisotropically. Hydrogen atoms are placed in geometrically calculated positions and are included in the refinement process using riding model. Putative 3D structures of the crystal are depicted in Fig. 3, wherein one CHX is paired with two LS molecules. It is believed that this may be related to the inhibition of CHX metabolism. The stoichiometric ratio between CHX and LS of 1:2 is consistent with the ¹H NMR spectral signals (Figure 1).

To further confirm the formation of the CHX-DS complex, the crystal is dissolved in methanol and analyzed by NMR and MS. Additionally, SC-XRD analysis is carried out at 100 K showing that the coordination complex CHX-DS crystallizes in triclinic P1 space group with the unit cell parameters a = 11.63(3) Å; b = 13.63(3) Å; c = 9.14(3) Å, and α = 70.24(10)°; β = 92.95(10)°; γ =89.76(2)°. The structural formula can be described as [C₂₂H₃₂N₁₀Cl₂]•[(C₁₂H₂₅O₄S)₂] with the asymmetric unit consisting of one molecule of doubly protonated chlorhexidine cation and two molecules of dodecyl sulfate anion. The structure comprises one CHX molecule surrounded by two DS molecules, whereby the biguanidine moieties of the CHX are symmetrically protonated by hydrogen transfer from the acidic sulfate group of the two DS molecules. These biguanidine moieties display a delocalization of the single and double bonds, as evident from the C-N bond lengths (1.313 to 1.357 Å). The CHX dications adopt a spiral conformation giving rise to U-shaped coils extending parallel to the a-axis. The CHX cations among the coils between the adjacent layers along the c-axis are anti to each other, giving rise to alternating layers of the CHX coils that are further involved in hydrogen bonding interactions to the sulfate anions of the DS molecules with one of the DS molecules being disordered. Each of the CHX cations is involved in hydrogen bonding interactions with the sulfate groups from three different DS molecules. Two sulfate groups form two hydrogen bonds with the biguanide groups on the outer periphery, and one sulfate forms three hydrogen bonds with the inner -NH and -NH2 groups of the biguanide moieties. The H-bonding ranges from 2.057 to 2.254 Å, indicating strong hydrogen bonding.42 The PXRD pattern calculated from the single crystal structure also matches quite well with the one obtained from the bulk sample, indicating bulk phase purity.

*FTIR experimental results:* The CHX-LS crystal is analyzed in FTIR spectrum. The peaks around 1400~1800 cm ⁻¹ are very similar to the SLS standard in red. The peaks around 800~1300 cm⁻¹ and 2000~3000 cm⁻¹ match the CHX standard. Therefore, the crystal contains both CHX and LS components.

Infrared spectra are collected using a Bruker Vertex 70 FTIR spectrometer (Bruker Optics, Billerica, MA) equipped with a GladiATR diamond ATR accessory (Pike technologies, Madison, WI). The spectra are acquired with a 4 cm-1 resolution in the 80-4000 cm-1 spectral range. All measurements were carried out at room temperature.

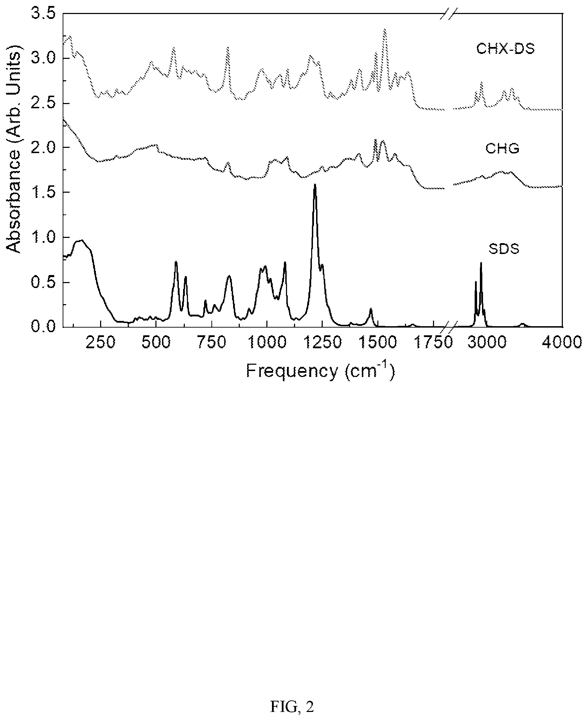

The FTIR absorption spectrum of the CHX-DS (or CHX-LS) crystal prepared in this work in comparison to those of the reference materials of SDS (sodium dodecyl sulfate, also sometimes referred to as sodium lauryl sulfate or SLS) and CHG (chlorhexidine gluconate) is shown in Figure 2 (spectra offset for clarity). Analysis of the CHX-DS infrared spectrum immediately reveals the presence of both CHX and SDS components in the sample. As an example, CHX bands corresponding to v(C=C), v(C=N), and δ(NH2) vibrations are clearly seen in the region above 1500 cm⁻¹, along with the N-H stretching modes of -NH, =NH, and NH2 functional groups in the 3000-3500 cm⁻¹ range. Similarly, the SDS component can be identified from a prominent cluster of bands in the 1200-1275 cm-1 region associated with vas(SO2) vibrations as well as intense νas/sym(CH₃/CH₂) vibrations in the 2800-3000 cm⁻¹ range. Evidence of both CHX and SDS vibrational characteristics in the FTIR spectrum of prepared crystal, combined with the fact that the absorption bands are significantly different in their shape and positions from the reference materials, suggests the formation of the salt between chlorhexidine and sodium dodecyl sulfate ions.

*LC-MS analytical results:* The acquired crystal is dissolved in methanol solvent and is injected to a Thermo Q Exactive hybrid Quadrupole-Orbitrap Mass Spectrometer instrument. The analyst CHX-LS solution is delivered with the mobile phase containing 50%MeOH-water solvent. MS detector is run in the positive mode. The peak at 505 m/z indicates pure chlorhexidine having the chlorine isotope pattern. It also gives the complexes formed between chlorhexidine with LS at 771 and 1059, corresponding to CHX-LS at 1:1 and 1:2 molar ratios, respectively. The MS of pure chlorhexidine and 1:1 ratio of chlorhexidine complex could come from 1:2 ratio of chlorhexidine fragmentation.

One proposed structure of the CHX-LS is Structure A 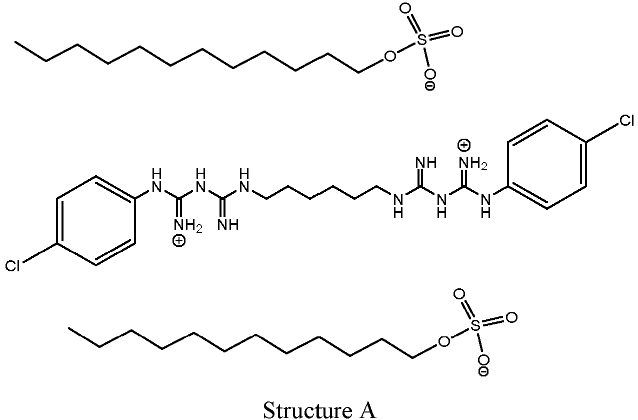 Alternatively, the sulfate moieties may pair with the same guanidinium group, as depicted in Figure 3 and Structure B below:

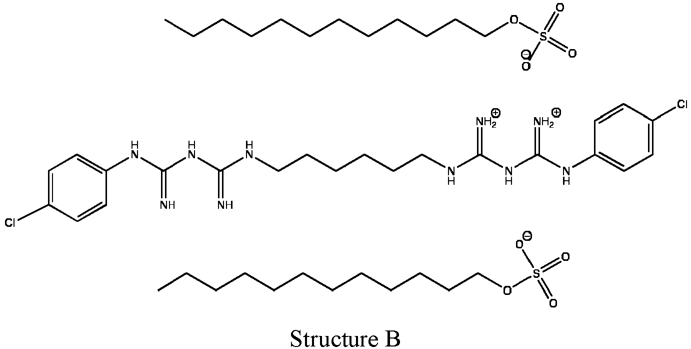

### Example 2: Formation of CHX-LS Salt From Different Concentrations of CHX and LX

A series of experiments testing the molar ratios of CHG:SLS required for formation of the CHX-LS complex. It is found that the CHX-LS complex prepared from CHG and SLS forms only at specific ratios of CHG:SLS, generally a molar ratio of at least 1:4 (≥ 0.25), e.g., 1:1 or 1:2, at a concentration of 0.12 wt % CHG. Where the amount of CHG relative to SLS is lower, e.g., at molar ratios of CHX:LS 1:5 or less (≤0.2), no detectable salt or precipitate is formed.

Existing oral care products generally use a very low CHG:SLS ratio. For example, CHG is typically used at 0.12% by weight while SLS is used at >1% by weight. A solution of 0.12% CHG by weight and 1% SLS by weight would have a molar ratio of CHG to SLS of only about 0.03, far less than the 0.25 or greater CHX/SLS molar ratio needed to form a precipitate starting with an aqueous solution of 0.12% CHG. Therefore, it is not expected that a solid salt precipitate would exist in such products.

### Example 3: Antibacterial efficacy and VSC reduction using CHX-LS salt

*In vitro* efficacy of chlorhexidine lauryl sulfate (CHX-LS) on reduction of volatile sulfur compounds (VSCs) is investigated using methyl mercaptan as the model sulfur compound. It is determined that CHX-LS exhibits superior antibacterial efficacy as compared to chlorhexidine gluconate (CHG) and greater reduction in volatile sulfur compounds (VSCs).

Clear solutions are prepared and used as precursors without purification. 10 wt.% and 20 wt.% solutions are prepared using sodium lauryl sulfate (SLS) and chlorhexidine gluconate (CHG), respectively, in absolute methanol. Both samples are sonicated to ensure complete dissolution. Sodium lauryl sulfate solution is added dropwise to the CHG solution. A crystalline "snow-flake" appearing material is formed after several minutes.

Methyl mercaptan (CH₃SH, CAS# 74-93-1) is a representative ingredient of volatile sulfur compound (VSC), which can be used as the marker for the quantitative measurement of mouth odor through gas chromatography-flame photometric detector technology. Sample preparation entails dissolution of the CHX-LS, SLS, CHX-HCl, and powders to a final concentration of 0.01 wt.%; additionally 0.01 wt.% of CHG solution is also prepared. Hydroxyapatite (HAP) is incubated with whole saliva to develop pellicles followed by the treatment of testing and control dentifrice slurries. After rinsing, the treated disks are transferred to headspace vials and incubated with VSC solution to mimic mouth odor (VSC) generation. The methyl mercaptan in headspace is measured through gas chromatography-flame photometric detector, and the results determine the product efficacy in mouth odor reduction.

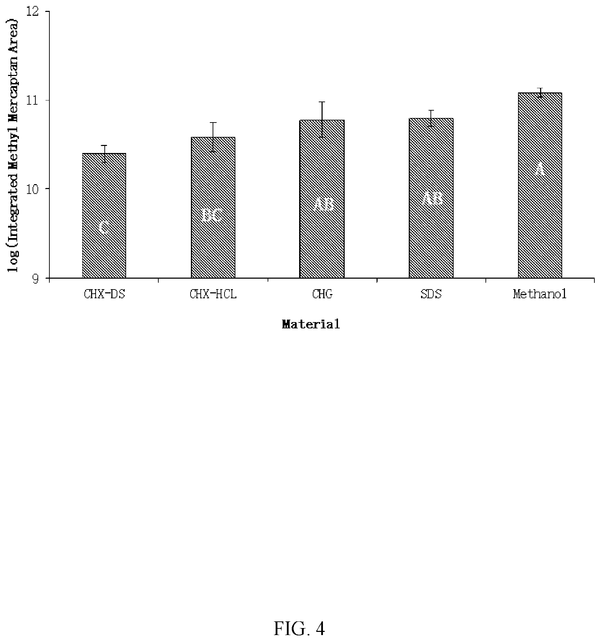

The effect of CHX-LS, CHG, CHX-HCl, SLS, and methanol on *in vitro* bacteria-generated volatile sulfur compounds (VSC), conducted by methyl mercaptan gas chromatography (GC) headspace measurement, is illustrated in Fig. 4. Statistical grouping, calculated using the Tukey method and a 95.0% confidence interval indicates that CHX-LS and CHX-HCl outperform other test materials for malodor-causing VSCs. In Fig. 4, the letters represent statistical groups. Means that do not share a letter are significantly different. The lower data means the better VSC reduction efficacy. CHX-LS has significantly better methyl mercaptan reduction efficacy than CHG and SDS. There is no significant difference between CHG, SLS and methanol.

This study shows that CHX-LS exhibits superior VSC reduction efficacy as compared to CHG. These data indicate CHX-LS is a viable and effective antimicrobial agent for malodor reduction in dentifrices and oral rinses.

## Claims

1. A solid salt of chlorhexidine lauryl sulfate, wherein the solid salt of chlorhexidine lauryl sulfate is:
(i) in crystalline form; or
(ii) in amorphous form.

2. The solid salt of chlorhexidine lauryl sulfate of claim 1 wherein a molar ratio between chlorhexidine (CHX) and lauryl sulfate (LS) is 1:4 to 1:1, or preferably 1:2.

3. An oral care composition comprising chlorhexidine lauryl sulfate wherein a molar ratio between chlorhexidine (CHX) and lauryl sulfate (LS) is 1:4 to 1:1, or preferably 1:2.

4. The oral care composition of claim 3 wherein the chlorhexidine lauryl sulfate is added to the other formulation excipients as a pre-formed solid salt.

5. The oral care composition of claim 3 or 4 wherein the chlorhexidine lauryl sulfate is added to water and other formulation excipients as a pre-formed solid salt.

6. The oral care composition of any one of claims 3-5 wherein the chlorhexidine lauryl sulfate is formed in situ by combining a solution of sodium lauryl sulfate with chlorhexidine wherein the molar ratio between chlorhexidine (CHX) and lauryl sulfate (LS) is 1:4 to 1:1, or preferably 1:2.

7. The oral care composition of any one of claims 3-6, wherein the oral care composition is a mouthwash further comprising flavorings, sweeteners, humectants, and surfactants.

8. The oral care composition of any one of claims 3-6, wherein the oral care composition is a toothpaste further comprising flavorings, sweeteners, humectants, surfactants, and abrasives.

9. The oral care composition of any one of claims 3-8 wherein a concentration of chlorhexidine lauryl sulfate is 0.05% to 0.4% by weight, or preferably 0.25% by weight.

10. A method of stabilizing chlorhexidine in an aqueous solution comprising adding the solid salt of chlorhexidine lauryl sulfate of claim 1 or 2 to the aqueous solution.

11. The oral care composition of any one of claim 3-9 for use in treating gingivitis, prophylaxis of dental plaque and caries, treating yeast infections of the mouth, and/or protecting against infections following oral surgery and tooth extraction.

12. The oral care composition of any one of claims 3-9 for use in reducing halitosis and/or reducing volatile sulfur compounds in the mouth.

13. A disinfectant composition comprising chlorhexidine lauryl sulfate, water, and alcohol.

14. The disinfectant composition of claim 13 for use in disinfecting the skin before surgery and/or cleaning a wound.

15. A method for sterilization of surgical or dental instruments, comprising administering the disinfectant composition of claim 13 to the instruments.

## Patentansprüche

1. Festes Salz von Chlorhexidinlaurylsulfat, wobei das feste Salz von Chlorhexidinlaurylsulfat:
(i) in kristalliner Form vorliegt; oder
(ii) in amorpher Form vorliegt.

2. Festes Salz von Chlorhexidinlaurylsulfat nach Anspruch 1, wobei das Molverhältnis zwischen Chlorhexidin (CHX) und Laurylsulfat (LS) 1:4 bis 1:1 oder vorzugsweise 1:2 beträgt.

3. Mundpflegezusammensetzung, umfassend Chlorhexidin-Laurylsulfat, wobei das Molverhältnis zwischen Chlorhexidin (CHX) und Laurylsulfat (LS) 1:4 bis 1:1 oder vorzugsweise 1:2 beträgt.

4. Mundpflegezusammensetzung nach Anspruch 3, wobei das Chlorhexidin-Laurylsulfat den anderen Formulierungshilfsstoffen als vorgebildetes festes Salz zugesetzt wird.

5. Mundpflegezusammensetzung nach Anspruch 3 oder 4, wobei das Chlorhexidin-Laurylsulfat Wasser und anderen Formulierungshilfsstoffen als vorgebildetes festes Salz zugesetzt wird.

6. Mundpflegezusammensetzung nach einem der Ansprüche 3 bis 5, wobei das Chlorhexidinlaurylsulfat in situ durch Vereinigen einer Natriumlaurylsulfat-Lösung mit Chlorhexidin gebildet wird, wobei das Molverhältnis zwischen Chlorhexidin (CHX) und Laurylsulfat (LS) 1:4 bis 1:1 oder vorzugsweise 1:2 beträgt.

7. Mundpflegezusammensetzung nach einem der Ansprüche 3 bis 6, wobei die Mundpflegezusammensetzung ein Mundwasser ist, das ferner Aromastoffe, Süßstoffe, Feuchthaltemittel und Tenside enthält.

8. Mundpflegezusammensetzung nach einem der Ansprüche 3 bis 6, wobei die Mundpflegezusammensetzung eine Zahnpasta ist, die ferner Aromastoffe, Süßstoffe, Feuchthaltemittel, Tenside und Abrasivstoffe enthält.

9. Mundpflegezusammensetzung nach einem der Ansprüche 3 bis 8, wobei die Konzentration an Chlorhexidinlaurylsulfat 0,05 bis 0,4 Gew.-% oder vorzugsweise 0,25 Gew.-% beträgt.

10. Verfahren zur Stabilisierung von Chlorhexidin in einer wässrigen Lösung, umfassend das Hinzufügen des festen Salzes von Chlorhexidinlaurylsulfat gemäß Anspruch 1 oder 2 zu der wässrigen Lösung.

11. Mundpflegezusammensetzung nach einem der Ansprüche 3 bis 9 zur Verwendung bei der Behandlung von Gingivitis, zur Prophylaxe von Zahnbelag und Karies, zur Behandlung von Hefepilzinfektionen im Mund und/oder zum Schutz vor Infektionen nach oralchirurgischen Eingriffen und Zahnextraktionen.

12. Mundpflegezusammensetzung nach einem der Ansprüche 3 bis 9 zur Verwendung bei der Verringerung von Mundgeruch und/oder zur Verringerung flüchtiger Schwefelverbindungen im Mund.

13. Desinfektionszusammensetzung, umfassend Chlorhexidinlaurylsulfat, Wasser und Alkohol.

14. Desinfektionszusammensetzung nach Anspruch 13 zur Verwendung bei der Desinfektion der Haut vor einer Operation und/oder der Reinigung einer Wunde.

15. Verfahren zur Sterilisation von chirurgischen oder zahnärztlichen Instrumenten, umfassend das Aufbringen der Desinfektionszusammensetzung nach Anspruch 13 auf die Instrumente.

## Revendications

1. Sel solide de chlorhexidine lauryl sulfate, dans lequel le sel solide de chlorhexidine lauryl sulfate est :
(i) sous forme cristalline ; ou
(ii) sous forme amorphe.

2. Sel solide de chlorhexidine lauryl sulfate selon la revendication 1, dans lequel un rapport molaire entre la chlorhexidine (CHX) et le lauryl sulfate (LS) est de 1:4 à 1:1, ou de préférence de 1:2.

3. Composition de soins bucco-dentaires comprenant du chlorhexidine lauryl sulfate dans laquelle un rapport molaire entre la chlorhexidine (CHX) et le lauryl sulfate (LS) est de 1:4 à 1:1, ou de préférence de 1:2.

4. Composition de soins bucco-dentaires selon la revendication 3, dans laquelle le chlorhexidine lauryl sulfate est ajouté aux autres excipients de formulation sous la forme d'un sel solide préformé.

5. Composition de soins bucco-dentaires selon la revendication 3 ou 4, dans laquelle le chlorhexidine lauryl sulfate est ajouté à de l'eau et à d'autres excipients de formulation sous la forme d'un sel solide préformé.

6. Composition de soins bucco-dentaires selon l'une quelconque des revendications 3 à 5, dans laquelle le chlorhexidine lauryl sulfate est formé in situ en combinant une solution de lauryl sulfate de sodium avec de la chlorhexidine, dans laquelle le rapport molaire entre la chlorhexidine (CHX) et le lauryl sulfate (LS) est de 1:4 à 1:1, ou de préférence de 1:2.

7. Composition de soins bucco-dentaires selon l'une quelconque des revendications 3 à 6, dans laquelle la composition de soins bucco-dentaires est un bain de bouche comprenant en outre des arômes, des édulcorants, des humectants et des tensioactifs.

8. Composition de soins bucco-dentaires selon l'une quelconque des revendications 3 à 6, dans laquelle la composition de soins bucco-dentaires est une pâte dentifrice comprenant en outre des arômes, des édulcorants, des humectants, des tensioactifs et des abrasifs.

9. Composition de soins bucco-dentaires selon l'une quelconque des revendications 3 à 8, dans laquelle une concentration de chlorhexidine lauryl sulfate est de 0,05 % à 0,4 % en poids, ou de préférence de 0,25 % en poids.

10. Procédé de stabilisation de la chlorhexidine dans une solution aqueuse comprenant l'ajout du sel solide de chlorhexidine lauryl sulfate selon la revendication 1 ou 2 à la solution aqueuse.

11. Composition de soins bucco-dentaires selon l'une quelconque des revendications 3 à 9, pour une utilisation dans le traitement de la gingivite, la prophylaxie de la plaque dentaire et des caries, le traitement des infections à levures de la bouche et/ou la protection contre les infections après une chirurgie buccale et une extraction dentaire.

12. Composition de soins bucco-dentaires selon l'une quelconque des revendications 3 à 9, pour une utilisation dans la réduction de l'halitose et/ou la réduction des composés soufrés volatils dans la bouche.

13. Composition désinfectante comprenant du chlorhexidine lauryl sulfate, de l'eau et de l'alcool.

14. Composition désinfectante selon la revendication 13 pour une utilisation dans la désinfection de la peau avant une chirurgie et/ou le nettoyage d'une plaie.

15. Procédé de stérilisation d'instruments chirurgicaux ou dentaires, comprenant l'application de la composition désinfectante selon la revendication 13 sur les instruments.
